# EUROPEAN PATENT SPECIFICATION

(11) **EP 3 000 872 B1**
(45) Date of publication and mention of the grant of the patent: **18.09.2019**
(21) Application number: 14461573.9
(22) Date of filing: 26.09.2014
(51) Int. Cl.: C12N 1/20, B01D 53/52, B01D 53/85, C10L 3/08, C10L 3/10

(54) **Microbiological medium for a microbiological consortium applied in the technology of biological purification of biogas**
Microbiologisches Medium für ein mikrobiologisches Konsortium angewendet in biologischer Biogasreinigungstechnologie
Milieu de culture microbiologique d'un consortium microbiologique appliqué dans la technologie de purification biologique de biogaz

(43) Date of publication of application: 30.03.2016
(73) Proprietor: Politechnika Lódzka, 90-924 Lódz (PL)
(72) Inventor: Zieminski, Krzysztof, 93-355 Lódz (PL)
(74) Representative: Rumpel, Alicja

(56) References cited:
- EP-A1- 2 460 883
- EP-A1- 2 767 585
- US-A1- 2001 006 809
- US-A1- 2013 115 672
- ZILOUEI H ET AL: "Two-phase partitioning bioreactor for the biodegradation of high concentrations of pentachlorophenol using Sphingobium chlorophenolicum DSM 8671", CHEMOSPHERE, vol. 72, no. 11, 13 June 2008 (2008-06-13) , pages 1788-1794, XP023178208, ISSN: 0045-6535, DOI: 10.1016/J.CHEMOSPHERE.2008.04.035
- JIANG R ET AL: "Nitric oxide removal from flue gas with a biotrickling filter using Pseudomonas putida", JOURNAL OF HAZARDOUS MATERIALS, vol. 164, no. 2-3, 26 August 2008 (2008-08-26), pages 432-441, XP026028005, ISSN: 0304-3894, DOI: 10.1016/J.JHAZMAT.2008.08.058
- TICHY R ET AL: "Oxidation of biologically-produced sulphur in a continuous mixed-suspension reactor", WATER RESEARCH, vol. 32, no. 3, 1 March 1998 (1998-03-01), pages 701-710, XP004107541, ISSN: 0043-1354, DOI: 10.1016/S0043-1354(97)00247-9
- MA ET AL: "Removal of H2S in waste gases by an activated carbon bioreactor", INTERNATIONAL BIODETERIORATION AND BIODEGRADATION, vol. 57, no. 2, 1 March 2006 (2006-03-01), pages 93-98, XP005321306, ISSN: 0964-8305, DOI: 10.1016/J.IBIOD.2005.10.010
- CARDONA I C ET AL: "Biodesulfurization of two Colombian coals with native microorganisms", FUEL PROCESSING TECHNOLOGY, vol. 90, no. 9, 1 September 2009 (2009-09-01), pages 1099-1106, XP026448431, ISSN: 0378-3820
- GOMEZ J M ET AL: "Kinetic equation for growth of Thiobacillus ferrooxidans in submerged culture over aqueous ferrous sulphate solutions", JOURNAL OF BIOTECHNOLOGY, vol. 48, no. 1, 18 July 1996 (1996-07-18), pages 147-152, XP004037044, ISSN: 0168-1656, DOI: 10.1016/0168-1656(96)01504-0

## Description

The invention concerns a microbiological culture medium suitable for culturing of a biogas purifying consortium of microorganisms, in the presence of aerobic forms of nitrogen, in a biofilter with biological filling.

Biogas is generated in result of an activity of anaerobic bacteria, causing degradation of organic substances. Biogas mixture usually contains approximately 55-85% of methane (CH₄), 30-45% of carbon dioxide C0₂), 0.015% of hydrogen sulphide (H₂S) plus low concentrations of nitrogen, oxygen and water vapour. Biogas purification from unnecessary substances considerably increases its calorific value. The need of biogas desulphurisation is also associated with the necessity to eliminate negative effects of H₂S on biogas combustion systems, protection of engines of electric power generators, and prevention of boilers and boiler burners from corrosion. Sulphated biogas combustion results in enhanced emissions of sulphur dioxide, which is responsible for acid rains. At the same time, there is a need to reduce emissions of H₂S as a component with strong toxic effects, highly detrimental for human health and the environment.

Technologies with no burdens for the natural environment are preferred both in biogas production and use,. However, biogas pre-treatment to achieve a composition suitable for gas networks by means of conventional methods, which are based mainly on active carbon sorption properties, is a cumbersome and expensive task. For the above-mentioned reasons, it is necessary to develop biological methods of biogas purification, as an alternative to chemical processes. They provide an attractive option for the low capital outlays and the lack of negative effects on the natural environment. The main requirements of the biotechnological methods of desulphurization of biogas include having appropriate, specially selected microorganisms plus the knowledge of conditions in which they may be effectively used. Actually , the key problem is the lack of highly effective methods of biological biogas purification on an industrial scale. Another problem, associated with the application of biological methods of biogas purification, is the presence of elemental sulphur in the installation used for desulphurisation. Sulphur compounds are oxidised to elemental sulphur, which is then deposited in the system and has to be regularly removed.

The actual solutions in the field of biological biogas purification are based mainly on a delivery of wastewater to treatment reactors, which serves as a source of carbon, nitrogen and biogenic elements for microorganisms. An example of such a solution has been known from Patent Description No. WO2012061933, where the organic substrate consists of animal, agricultural, municipal, agricultural- food-processing, industrial wastes or a mixture thereof. Another example of application of waste materials as a culture medium for the microorganisms removing H₂S from biogas is presented in Patent Description No. WO2008131034. In such systems heterotrophic microorganisms, which do not demonstrate any ability of binding CO₂, dominate as a result of natural selection, thus being unable to remove CO₂ from biogas.

In another example of hydrogen sulphide removal from biogas, known from Patent Description No.DE3912418, the microbiological culture medium contains NaHCO₃ (0.8 mg/L), NH₄Cl (0.1 mg/L), MgSO4x7H₂O (0.08 mg/L), CaCl₂x2H₂O (0.01 mg/L), K2HPO4 (0.2 mg/L), citric acid (0.05 mg/L), FeCl₃x6H₂O (0,0005 mg/L), vitamin B12 (0.0002 mg/L), yeast extract (0,05 mg/L), Na₂Sx9H₂O (0,25 mg/L), sodium acetate (0,2 mg/L) and tap water. As the culture medium contains in its composition a source of carbon the microorganisms will not assimilate it from CO₂.

Patent Description No. EP1604727 presents a process of biogas desulphurisation in aerobic conditions using nutritional components in the medium, such as (NH₄)₂SO₄, KCI, K₂HP0₄, MgSO₄ and Ca(N0₃)₂. In this biogas purification process, a chemical reaction provides elemental sulphur, which is then deposited in the instalation.

In the publication by Ma et al. (Ma et al., 2006. International Biodeterioration & Biodegradation 57:93-98) H₂S was removed from waste gases by an activated carbon bioreactor with the help of a pure culture of T. denitrificans. The ATCC medium contained 1.2 g Na₂HPO₄, 1.8 g KH₂PO₄, 0.1 g MgSO₄x7H₂O, 0.1 g (NH₄)₂SO₄, 0.03 g CaCl₂, 0.02 g FeCl₃x6H₂O, 0.02 g MnSO₄x4H₂0, 10 g Na₂S₂O₃x5H₂O, 5 g NaHCO₃ x 5H₂O, 0.5 g NaHCO₃ and 5 g KNO₃ per 1L of the culture medium. The concentration of Ca²⁺ ions in the culture medium is lower than in the medium of the present invention. Additionally, the ATCC medium lacks many of the trace elements, which are present in the medium of the present invention.

In the publication of Cardona et al. (Cardona et al., 2009. Fuel Processing Technology 90:1099-1106) bidesulfurization of coal was performer by a consortium of microorganisms, derived from a coal mine. The modified 9K medium had the following composition: 3 g (NH₄)₂SO₄, 0.5 g MgSO₄ x 7H₂O, 0.5 g K₂HPO₄, 0.1 g KCI, 0.01 g Ca(NO₃)₂ per 1L of the culture medium. The concentration of Ca²⁺ ions in the culture medium is lower than in the medium of the present invention. There is also no iron source. Additionally, the modified 9K medium lacks all of the trace elements, which are present in the medium of the present invention.

Patent application EP2767585 reveals a microbiological metod of removal of H₂S under anoxic conditions by means of a biofilter loaded with a biological filter bed, sprinkled with a mineral medium contianing nitrogenous salts of alkali metals of the I and II group, wherein the concentration of nitrogen ions in the medium ranges from 20 to 2500 mg/l. The concentration of nitrogen ions in the medium according to the present invention is lower than in the medium described in EP2767585. Additionally, no other components of the culture medium according to EP2767585 have been revealed, in particular no iron source or trace elements sources.

Cations or anions present in a culture medium do affect the strength of the biofilm and the composition of the microbiological consortium purifying the biogas, and changing these is expected to shift or change the biodiversity of the consortium, and therefore its functionality, as it often happens in environmental biofilms, e.g. during the incidents of saltwater intrusion into biological wastewater treatment plants (exchange of Ca²⁺ for Na⁺), or chemical shocks in attached-growth biofilm processes.

A microbiological culture medium for culturing of a biogas purifying consortium of microorganisms, according to this invention is composed of KH₂PO₄ 100 g/500 L, MgCl₂x6H₂O 50 g/500 L, Ca(NO₃)₂x4H₂O 50 g/500 L and FeSO₄x7H₂O 40 g/500 L and trace elements ZnSO₄ 5.0 g/500 L, MnCl₂ 10.0 g/500 L, CoCl₂ 3.0 g/500 L, CuSO₄ 2.0 g/500 L and (NH₄)₂MoO₄ 1.0 g/500 L.

Culture medium according to this invention is used for sprinkling biological sediments with immobilized microorganisms, which degrade hydrogen sulphide. The applied microorganisms include bacteria isolated from their natural habitat. The bacteria form a specific biofilm on the packing surface, processing the absorbed hydrogen sulphide into elemental sulphur or sulphates. Genetic analyses allowed to determine the microbiological consortium in the biofilm at various phylogenetic levels, the bacteria belonging to Alphaproteobacteria, Betaproteobacteria and Gammaproteobacteria classes. The level of biodiversity and the dominating bacteria are very important for the process efficiency. Selection of microorganisms, forming a specific biofilm, was possible as a result of the characteristic culture medium composition according to this invention and an appropriate filtration system. The volumetric flow intensity of sprinkling of the biological sediment with the microbiological culture medium preferably ranges within 5 L/h to 500 L/h per one cubic meter of the filling. It is preferable that pH of the culture medium is in the range from 5 to 8, preferably not below 5.5 and not above 7.0-7.5.

The advantage of this invention is 97-100% H₂S reduction efficiency under industrial conditions, allowing to achieve biogas parameters comparable with the natural gas parameters. What is more, as there is no carbon source present in the culture medium, the microorganisms collect it from CO₂, which contributes to removal of this ballast material from biogas. Another advantage of this invention is a minimised sulphur production in the biogas purification process and limited deposit volumes in the process installation. The process does not lead to a pH drop, which eliminates the need for later pH adjustment. The purified biogas is not diluted with air, which eliminates the danger of explosive oxygen and biogas mixture formation, while reducing the costs of the installation maintenance.

The subject of the invention has been explained in detail in the application example.

### Example: preparation of 500 L of the culture medium

A 600 L container was filled with tap water, poured in a continuous stream. While pouring the tap water, the following solutions were subsequently added: 40 g of FeSO₄x7H₂O, solved in 1 L of tap water, 50 g of MgCL₂x6H₂O, solved in 1 L of tap water and 100 g of KH₂PO₄, solved in 1 L of tap water. When approximately 400 L of water was poured, a solution of 50 g Ca(NO₃)₂x4H₂O was added plus a mixture of trace elements ZnSO₄ -5.0 g,, MnCl₂ - 10.0 g, CoCl₂ - 3.0 g, CuSO₄ - 2.0g (NH₄)₂MoO₄ - 1.0 g and water filling was continued up to the nominal volume. The whole volume was mechanically stirred for approximately 15 minutes.

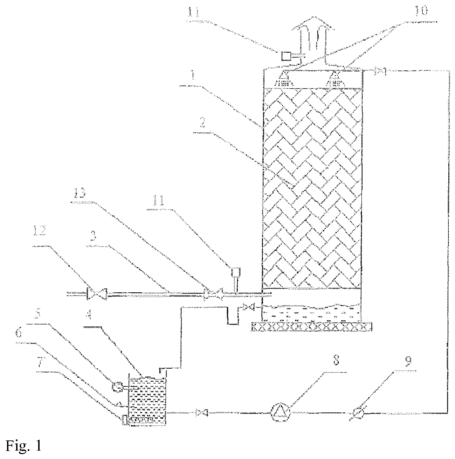

This invention employed a system based on a sprinkled biofilter, working as an element of an industrial installation. Contaminated gas was delivered from the bottom of the system, while a recirculating culture medium sprinkled the column packing from the top in a volume of 18 L/h. The microorganisms, forming a consortium of microorganisms, were isolated from natural environments, rich in sulphur. The consortium, immobilized on the column filling, was able to efficiently purify biogas, . The installation for biogas purification has been illustrated in the drawing, where fig. 1 presents its structure. Biogas, produced in the process of methane fermentation, was pumped into the pipeline (3) and then to the biological desulphurisation system. The incomming biogas volume was controlled with a flow control (13). H₂S measurement and recording, both at inlet and outlet from the biofilter, was performed by H₂S concentration sensors (11) and confirmed by chromatography. A biological system, consisting of a porous filtration material (2), enabling formation of a specific biofilm with an activity level ensuring biogas purification, was placed in the biofilter column (1). The biofilter system was sprinkled from the top (10) with the culture medium in a volume of 18 L/h. The culture medium, dripping through the biofilter down to the column bottom, was drained into the tank (4). The culture medium was then returned to the system with a pump (8), flowing through a flow meter (9) up to the sprinklers (10). The culture medium tank (4) was equipped with a heater (7) with a temperature sensor (5) to maintain constant temperature in the tank, as well as a culture medium level sensor (6). Biogas flow into the installation was regulated by the valve (12). Desulphurisation efficiency reached 98-99%.

## Claims

1. A microbiological culture medium suitable for culturing of a biogas purifying consortium of microorganisms, **characterised in that** it is composed of KH₂PO₄ 100 g/500 L, MgCl₂x6H₂O 50 g/500 L, Ca(NO₃)₂x4H₂O 50 g/500 L and FeSO₄x7H₂O 40 g/500 L and trace elements ZnSO₄ 5.0 g/500 L, MnCl₂ 10.0 g/500 L, CoCl₂ 3.0 g/500 L, CuSO₄ 2.0 g/500 L and (NH₄)₂MoO₄ 1.0 g/500 L.

2. The microbiological culture medium, according to claim 1, **characterised in that** its pH is in the range from 5 to 8.

## Patentansprüche

1. Mikrobiologisches Kulturmedium, das zur Kultivierung eines Biogas-Reinigungskonsortiums von Mikroorganismen geeignet ist, **dadurch gekennzeichnet, dass** es aus KH₂PO₄ (100 g/500 L), MgCl₂x6H₂O (50 g/500 L), Ca(NO₃)₂x4H₂O (50 g/500 L) und FeSO₄x7H₂O (40 g/500 L) und Spurenelemente ZnSO₄ (5.0 g/500 L), MnCl₂ (10.0 g/500 L), CoCl₂ (3.0 g/500 L)), CuSO₄ (2.0 g/500 L) and (NH₄)₂MoO₄(1.0 g/500 L)

2. Mikrobiologisches Kulturmedium nach Anspruch 1, **dadurch gekennzeichnet, dass** sein pH-Wert im Bereich von 5 bis 8 liegt.

## Revendications

1. Milieu de culture microbiologique adapté à la culture d'un consortium de micro-organismes épurateurs de biogaz, **caractérisé en ce qu'**il est composé de KH₂PO₄ (100 g/500 L), MgCl₂x6H₂O (50 g/500 L), Ca(NO₃)₂x4H₂O (50 g/500 L) et FeSO₄x7H₂O (40 g/500 L) et oligo-éléments ZnSO₄ (5.0 g/500 L), MnCl₂ (10.0 g/500 L), CoCl₂ (3.0 g/500 L)), CuSO₄ (2.0 g/500 L) and (NH₄)₂MoO₄(1.0 g/500 L)

2. Milieu de culture microbiologique selon la revendication 1, **caractérisé en ce que** son pH est compris entre 5 et 8.
